# EUROPEAN PATENT APPLICATION

(11) **EP 1 064 960 A2**
(43) Date of publication of application: **03.01.2001**
(21) Application number: 00113483.2
(22) Date of filing: 26.06.2000
(51) Int. Cl.: A61M 1/16, A61M 1/34

(54) **Hemodialysis assembly and method**

(30) Priority: 28.06.1999 US 329269
(71) Applicant: DENCO, INC., Wilmington, DE 19809 (US)
(72) Inventor: Spencer, Dudley W.C., Wilmington, DE 19809 (US)
(74) Representative: Wagner, Karl H., Dipl.-Ing.

(57) **Abstract**

A hemodialysis system and method of operation includes providing a plasma separator, such as a centrifuge in a process control to remove the plasma fluid from the red and white blood cells so that only the plasma fluid is subjected to the contaminant removal in a downstream dialyzer. This shortened by half the time required to perform a hemodialysis treatment a compared to conventional systems.

## Description

### Background of the Invention

Hemodialysis was advanced as a life sustaining therapy for end-stage renal failure, when in about 1925 it was discovered that a thin film of re-constituted cellulose (celophane®) had the ability to separate chemical substances while in solution by means of their unequal diffusion through a permeable membrane. This phenomenon performed particularly well with whole blood to differentially separate out Urea molecules. Over the years there have been many changes to incrementally improve the Hemodialysis process. The fundamentals, however, remain the same. Hemodialysis essentially mimics the kidney function of processing whole blood.

Whole blood is made up of three major components: a) red blood cells, b) white blood cells, c) plasma fluid. Since the toxins or Ureas to be removed are in the plasma solution portion of the whole blood, the cells just go along for the ride. The cells are also very fragile, so although they do not participate in the dialysis process directly, their presence dominates the rate at which hemodialysis can proceed. Generally, a hemodialysis process would take place over a six to eight hour time period for treatment and generally such treatments would be required 2 to 3 times per week. The dialyzer used is also patient specific.

It would be desirable if improvement could be made in the hemodialysis process, which greatly reduced the cost, and complexity: enough to effectively become a home based therapy.

### Summary of the Invention

An object of this invention is to provide an improved hemodialysis assembly capable of achieving the above desires.

A further object of this invention is to provide a hemodialysis method resulting from use of such assembly.

In accordance with this invention the whole blood removed from the patient is conveyed to a process control which includes a plasma separator, preferably a centrifuge, to separate the plasma from the red and white blood cells and platelets if necessary. The plasma is then removed from the process control and directed to a dialyzer where it is treated to remove contaminants and thus be purified. Alternatively, the plasma is collected in a bag or container for later purification. The cells are returned from the process control back to the patient. By removing the cells from the plasma, the process may be shortened in time since the cells are no longer present to adversely affect the process rate.

In the preferred practice of the invention sterile connection devices are used at various locations to facilitate the addition and removal of the various components or modules from the system in a quick, totally contained and sterile manner.

### Brief Description of the Drawings:

Figure 1 is a schematic diagram representing a currently used hemodialysis process of the prior art;
Figures 2 and 3 are schematic diagrams showing alternative hemodialysis assemblies in accordance with this invention; and
Figure 4 is a schematic diagram showing sterilizing or removal of contaminants from the plasma in accordance with this invention.

### Detailed Description

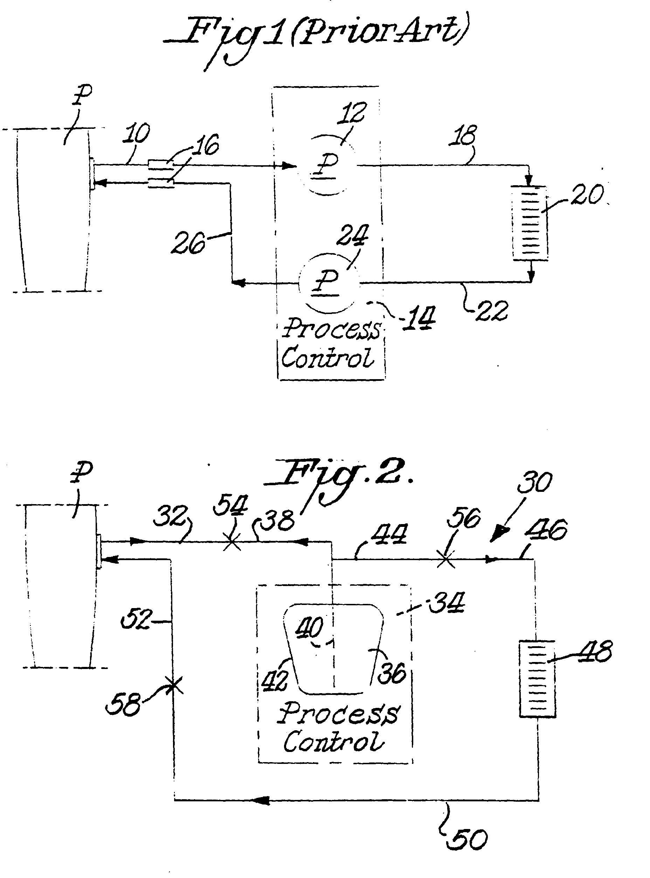

Figure 1 represents a current prior art hemodialysis assembly. As shown therein a tube 10 is connected to the chest or arm of a patient P. A pump 12 in process control 14 withdraws whole blood from the patient P into tube 10. The whole blood passes through a bacteria filter 16 then removed form the process control 14 through tube 18 which leads to a dialyzer 20. Dialyzer 20 may be, for example, a component commercially available from such companies as Baxter, Gambro and Fresenius.
The whole blood is treated to remove contaminants in dialyzer filter 20 and then conveyed through tube 22 by means of pump 24 in process control 14. The blood is then returned to the patient P through tube 26, which incorporates a further bacteria filter 16.

The dialyzer removes Urea molecules from the plasma via Donan dialysis. Because the cells are present, however, this process is hazardous in particular for the red cells, which may cause cell breakdown and fracture. To avoid the problems the hemodialysis conventionally used is slow, because of the need to protect the fragile red blood cells from damage. The use of bacteria filters is also a disadvantage in that such filters are not completely effective and also extend the time involved in the process. Such conventional process also incorporates a low pressure pump in the process control and utilizes low pressure in the dialyzer. The process described in Figure 1 must be performed in a clinical setting which requires the patient to visit the clinics about 2 to 3 times per week for dialysis treatment that last 6 to 8 hours per visit.
The inconvenience, loss of productive time for the patient, and the cost of clinic supervision all contribute to excessive costs for routine health care.

Figure 2 shows system 30 in accordance with this invention. As shown therein, whole blood would be removed from the patient P through tubes 32 and 38 and conveyed into process control 34.
In accordance with this invention, process control 34 includes a plasma separator 36 for removing the plasma from the cells. A preferred separator is a centrifuge. For example, Haemonetics has used a continuous centrifugal bowl to separate cells from plasma. This system is referred to as apheresis and has been in use since about 1950. The centrifuge takes advantage of the different densities of the plasma as compared to the blood cells. During operation with the centrifuge, the cells collect on the outer wall of the centrifuge bowl 42 because of their density while the plasma which is more water like or fluid remains in the center of the bowl. Platelets can also be removed from the plasma by a second "hard" spin if necessary.

Thus, the anticoagulated whole blood would be conveyed from tube 32 into upstream tube 38 and then pumped down the feed tube 40 to enter the bowl 42 at the bottom of centrifuge 36. The centriftigal force spins the denser cellular components to the outside leaving the plasma in the inner band. When the bowl is full, plasma flows out of the centrifuge 36 through feed tube 40 then through effluent tube 44. From tube 44 the plasma flows through downstream tube 46 into dialyzer 48 where contaminants are removed and the plasma is purified. The re-newed plasma is returned to the patient through return tubes 50, 52.

After the plasma has been removed from the centrifuge 36 the centrifuge bowl stops spinning and the blood cells are pumped through the feed tube 40 and back through tube 38 and tube 32 to be returned to the patient. Thus, the processing in the dialyzer is only of plasma and not of the blood cells.

The process resulting from use of system 30 is such that it would be possible for a patient to perform hemodialysis at home, at night in no more than four hours.

One of the factors that permits system 30 to achieve its substantial improvements over the conventional system is the use of sterile connection devices 54, 56, 58 at various locations in the system. Such sterile connection devices may be of the type described in U.S. Patent Nos. 4,793,880, 5,141,592, 5,256,226, 5,279,685 and 5,674,333 all details of which are incorporated herein by reference thereto.

As used herein the term "sterile connection device" is intended to refer to a device having a pair of aligned spaced tube holders, each of which has at least one tube receiving channel and includes a heated wafer located to pass between the spaced tube holders for heating plastic tubes placed therein. The connection or separation of such plastic tube is done in a sterile manner.

The ability to include a sterile connection device as part of the hemodialysis system assures that the hemodialysis processes can remain totally contained and sterile.

The use of such sterile connection devices 54, 56, 58 permits various components of the system to be added or replaced in a quick and efficient and particularly in a sterile manner. For example, the tubes 32 and 52 may remain essentially permanently connected to the patient and may be connected to the system 30 by use of sterile connection devise 54 which joins tube 38 to tube 32 and by sterile connection device 58 which connects tubes 50 and 52. Similarly, tubes 50 and 52 may be disconnected and sealed by sterile connection device 58. The process control 34 may be installed or removed from the system by use of sterile connection device 54 which selectively joins or separates tubes 32 and tubes 38. Thus, when the process control 34 is to be installed sterile connection device 54 connects tubes 32 and 38 and sterile connection device 56 connects tubes 44 and 46.

Air enters the system through device 56. The reverse process is used to separate the tubes and remove the process control 34 after treatment has been completed. Accordingly, process control 34 is a removable module.

Sterile connection device 58 is used to selectively connect or separate tubes 50 and 52 which in conjunction with sterile connection device 56 permits the installation or removal of dialyzer 48 from the system. Accordingly, dialyzer 48 may also be a removable module. The dialyzer 48 may also be a commercially available component such as marketed by Baxter, Gambro and Fresenius. In this embodiment the dialyzer module, however, is patient specific.

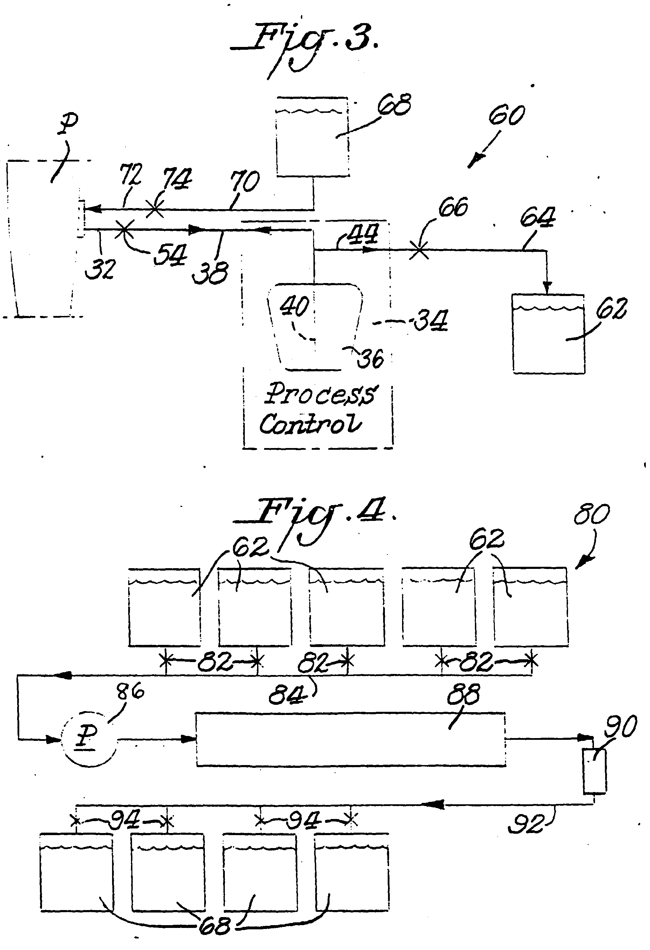

Figure 3 illustrates an alternative system 60 in accordance with this invention. As shown therein, the system 60 includes the same tube arrangement 32, 38, 40 and 44, the same process control 34 with the centrifuge 36 and feed tube 40 as well as sterile connection device 54 in the system 30. System 60 differs from system 30 in that the plasma removed from the patient is not returned to the patient. Instead, plasma from a different source 68 is fed to the patient. Thus, as with system 30, in system 60 whole blood in conveyed through tubes 32, 38 into separator 36 and the plasma is removed and fed through tube 44 after which the blood cells are returned through tubes 38 and 32 to the patient.
The plasma fed through tube 44 is then fed into a collection device such as a bag 62 by means of tube 64. A sterile connection device 66 permits the installation or removal of bag 62 and its tube 64 from the system. Air enters the system at device 66. While plasma is being collected in bag 62, sterile or purified plasma is fed from a separate bag 68 and through return tube 70 and then through tube 72 to the patient P. Sterile connection device 74 permits the selective installation and removal of bag 68 and its tube 70 from system 60. As is apparent process control 34 and containers 62, 68 are each installable/removable modules.

The system 60 of Figure 3 is particularly advantages since the plasma can be rendered non-patient specific while in the contaminated form. The plasma can be sterilized prior to dialyzing and the sterilized and contaminate reduced plasma is later returned to the patient. Thus, in the system 60, the plasma to be cleaned is simply collected in sterile bag 62 and when bag 62 is full the bag is sealed off, removed and replaced by a new empty sterile plasma bag. While this is being accomplished plasma from fresh bag 68 is simultaneously being returned to the patient. The plasma in bag 68 need not be plasma from the same patient.

Figure 4 schematically illustrates a system for sterilizing or removing contaminant from the plasma. As shown in Figure 4 the contaminant removal system 80 includes providing a plurality of bags 62 containing plasma that have been sterilized by heat treatment or radiation to kill viral and bacterial elements before it has been treated. By use of various sterile connection devices 82 the bags or collectors 62 are selectively installed or removed from inlet manifold or common tube 84. The plasma is removed under the influence of pump 86 and conveyed to plasma clean up center or dialyzer 88 which may be of the plate and frame type or may be of the hollow fiber type.

A suitable dialyzer may be a component marketed as PERMASEP®, which is marketed by the DuPont Company. In the 1960's this hollow fiber dialyzer was used to extract potable water from seawater. The system was used in arid lands with access to the sea.

This system is used for water purification, but its use has also been suggested for hemodialysis. The general operation of this type of dialyzer is to feed the liquid under pressure into a distributor tube that runs through the center of a hollow fiber bundle. As the liquid moves through the bundle around the fibers, pressure causes pure liquid to pass through the semi-permanent walls of the tiny fibers to their hollow cores leaving up to 99.2% of the contaminants behind.
The rejected contaminants remain outside the fiber walls and flow out through an outlet at the feed end of the permeator.

The use of sterile connection devices results in the systems being of a modular nature. For example, the system 30 shown in Figure 2 would include tubes 32 and 52, which would be generally permanently attached to the patient. A module is created by sterile welding devices 54, 56 which includes the intermediate tubes 38 and 44 and the process control 34. This module is connected to or removed from the system 30 by the sterile connection device 54 selectively connecting or disconnecting tubes 32 and 38 and by sterile collection device 56 selectively connecting and disconnecting tubes 44 and 46. A further module includes tubes 46 and 50 and the intermediate dialyzer 48 which is connected to or removed from the system by sterile collection devices 56 and 58. In this regard, tubes 44 and 46 are selectively connected to each other or disconnected by sterile collection device 56 while tubes 50 and 52 are selectively connected to or disconnected from each other by sterile collection device 58.

The modules are also formed in system 60 of Figure 3. Tubes 32 and 72 would be generally permanently connected to the patient. The supply container module which includes container or bag 68 and its tube 70 would be selectively connected to or disconnected from tube 72 by sterile collection device 74. Similarly, a module which includes process control 34 and its tubes 38 and 44 would be selectively connected to or disconnected to or removed from the system by sterile connection device 54 which connects or disconnects tubes 32 and 38 and by sterile connection device 66 which connects or disconnects tubes 44 and 64. Finally, a module is formed from collection bag 62 by means of sterile collection device 66 which selectively connects tubes 44 and 64 to each other or separates tubes 44 and 64 to each other or separates tubes 44 and 64 from each other.

Pump 86 is a high pressure pump operating, for example, at about 250 to 1000 lbs. Per square inch. Such high pressure pump assures the proper feeding of the contaminated plasma in the dialyzer 88. The spent or clean plasma is removed from dialyzer 88 through pressure reducer 90 into manifold 92. From its common supply manifold tube 92 the sterilized or cleaned plasma is fed into bags 68.

The containers 68 are selectively installed and removed from the system 80 and more particularly from its connection to tube 92 by means of sterile connection devices 94.

System 60 in addition to re-administering, sterilized, dialyzed plasma can also be used to suppress the onset of AIDS, for example, for people who are HIV positive. Viral invasion into the body uses the blood as the transport pathway to locate and enter cells. Once in a cell, they multiply and mature. When mature, they re-enter the blood pathways to find and enter cells as before. Viral infections once inside a cell are extremely difficult to eradicate. They are vulnerable, however, while in the blood stream. Using system 60 to sterilize the plasma fraction of the blood which contains the free floating viral free-agent; the re-infection highway can be cleared and under control. Although this is not a cure for viral infections that reside inside the cells, it does open a route to keep the proliferation of infection under control.

Methods of viral proliferation are under intense study to inactivate the viral infection once the virus has attached to cellular membranes, viral nucleic acid integrated into cellular DNA and other viral infected cells. Methods such as cell radiation, photosensitizing agents and drugs: All of these efforts are directed toward a cure: while commendable, this route is difficult because of rapid mutation of viral infections. The system 60 reduces the route of proliferation to a manageable, therapy mode. Holding the line, until a cure is found.

The use of sterile connection devices 82 and 94 are key to permitting multiple sets of containers 62 and 68 to be simultaneously processed.

The invention provides a number of distinct advantages over prior known hemodialysis systems and processes. As noted, the invention results in no cell damage, in reduced dialysis time, in overall reduction of hemodialysis costs, and in reduced trauma to patients.

Peritoneal dialysis has been cleared by the Health Care Regulatory Agency (FDA) for home care, this invention seeks to have hemodialysis also become a home care therapy.

To accomplish this, known and proven technologies are combined in such a way as to make this possible.
a) Total containment, sterile connection technology.
b) Cell-free dialyzing.
c) Cell-free processing of plasma to eliminate viral specie during plasma processing such as, HIV, HBV, HCV, or other viral infections.
For example patients who do not have End-stage renal failure, but need to combat the HIV viral attack of cells could benefit from the new protocol. Viral infections must use plasma as a transport means to find and attach themselves to cells. By keeping the viral population under control via plasma sterilization, (heat, radiation) the onset of AIDS could also be brought under control.

The protocol described in Figure 3 is a method of producing a plasma product that is disease-free and non-specific. The purified plasma could be re-bagged and dispensed for patient care, similar to protocols approved for peritoneal dialysis. In other words, the patient could be freed of the present archaic procedures of hemodialysis and like peritoneal dialysis patients, enjoy a more normal lifestyle: Not to mention the costs.

It should be noted that the objects and advantages of the invention may be attained by means of any compatible combination(s) particularly pointed out in the items of the following summary of the invention.

### SUMMARY OF THE INVENTION

A hemodialysis system comprising an upstream tube for connection to a patient for the removal of blood containing red blood cells and white blood cells and plasma fluid, said tube being in communication with a process control, said process control including separating the plasma fluid from the cells, said process control returning the cells to the patient through a blood cell return tube, a plasma collector, downstream tube leading from said process control to said plasma collector, a plasma supply container, and a plasma return tube leading from said plasma supply container, and a plasma return tube leading from said plasma supply container to the patient for conveying uncontaminated plasma fluid to the patient.
The system wherein said separating structure comprises a centrifuge.

The system wherein said supply container is also said collector.
The system wherein said collector is a dialyzer for removing contaminants from said plasma fluid and conveying purified plasma fluid to the patient.
The system including a first sterile connection device mounted at said upstream tube, a second sterile connection device mounted at said downstream tube, and a third sterile connection device mounted at said return tube.
The system wherein said process control and said supply container comprise separate modules, which are selectively removable, and installable into said system by means of said sterile connection device.
The system wherein said separating structure comprises a centrifuge.
The system wherein said plasma collector and said plasma supply container are separate containers.

The system including in combination therewith, a sterilizing assembly, said sterilizing assembly comprises a plurality of collection bags for containing contaminated fluid, such bag contents sterilized prior to dialyzing, a dialyzer, tubing connecting said collection container to with said dialyzer, a sterile connection device mounted at flow communication at said tubing for each of said collection container, a plurality of plasma supply containers communicating with said dialyzer for collecting sterilized plasma fluid from said dialyzer after contaminates have been removed from said plasma fluid, said supply containers being connected to said dialyzer by tubing, a sterile connection device for each of said supply containers mounted at said tubing, and one of said supply containers and one of said plasma collectors being said plasma collector and said supply container for said homodialysis system. A plasma sterilizing assembly comprising a plurality of plasma collection containers for containing contaminated plasma, a dialyzer for removing contaminants from the plasma, inlet tubing connecting said collection containers with said dialyzer, a sterile connection device for each of said supply containers mounted at said supply tubing for selectively installing and removing each of said supply containers from said assembly.
The assembly including a high pressure pump mounted at said inlet tubing, and a pressure reducer at said supply tubing.
A method of hemodialysis comprising mounting an upstream tube to a patient for the removal of whole blood containing red blood cells and white blood cells and plasma fluid from the patient, flowing the whole blood to a process control, separating the plasma fluid from the red blood cells by use of separating structure in the process control, flowing the plasma fluid to a plasma collector, returning the red blood cells and the white blood cells back to the patient, and supplying the patient with uncontaminated plasma from a supply container.
The method wherein the plasma fluid is separated from the cells by a centrifuge having a bowl which causes the cells to collect on the side of the centrifuge bowl while the plasma supply tubing for selectively installing and removing each of said supply containers from said assembly.

The method wherein the hemodialysis treatment is completed in no more than four hours.
The method wherein the same container which receives the plasma fluid from the centrifuge is also the plasma collector, and the container being a dialyzer removing contaminants from the plasma fluid.
The method wherein the plasma removed from the centrifuge is discharged into a plasma collector which is a different container than the plasma supply container.
The method wherein contaminants are removed from the plasma fluid by a plasma sterilizing assembly in which a plurality of plasma collectors containing contaminated plasma is mounted in flow communication by inlet tubing to a dialyzer removing the contaminates from the plasma fluid in the dialyzer, providing a plurality of supply containers in flow communication with the dialyzer by supply tubing, and collecting sterilized plasma fluid in the supply containers.

The method wherein a sterile connection device is provided for each of the of the plasma collectors at the inlet tubing for permitting the selective installation and removal of each plasma collector, and a sterile connection device being provided for each of the plasma supply containers at the supply tubing for permitting the selective installation and removal of each plasma supply container.
The method wherein at least one sterile connection device is mounted to the tubing to permit the selective cutting and welding of the tubing by the sterile connection device so components of the system used for the hemodialysis method could be selectively removed and installed in the system.

The method wherein viral elements are removed from the treated plasma to control viral proliferation.
The method wherein the method is used to reduce uncontrolled HIV spread within the patient to reduce HIV + becoming AIDS.
The method wherein the viral elements are removed by a heat or radiation treatment which kills the viral constituents.
The method wherein the method is performed as part of a home care treatment.
The method of claim 12 including separating platelets from the plasma fluid in the process control.
The assembly including a sterile connection device for each of said collection containers at said inlet tubing for selectively installing and removing each of said collection containers from said assembly.
A hemodialysis system for removing substances from blood cells comprising a supply of blood cells containing substances, a removal station, said removal station having a spinning structure for receiving the blood cells and substances to separate the blood cells from the substances, and a collection station downstream from the removal station for collecting the substances.

The system wherein said spinning structure is a centrifuge, and said collection station includes a filter.
A method of removing substances from blood cells comprising the steps of supplying the blood cells and substances to a spinning structure, spinning the spinning structure to separate the blood cells from the substances, conveying the substances to a downstream collection station, and collecting the substances.
The method wherein both plasma and other substances are separated from the blood cells in a centrifuge, and the blood cells are separated by being disposed against the wall of the centrifuge bowl as a result of the spinning, while the plasma and other substances remain in the interior of the centrifuge bowl.
The method wherein the other substances are removed from the plasma at the collection station by means of a filter.

## Claims

1. A hemodialysis system comprising an upstream tube for connection to a patient for the removal of blood containing red blood cells and white blood cells and plasma fluid, said tube being in communication with a process control, said process control including separating structure for separating the plasma fluid from the cells, said process control returning the cells to the patient through a blood cell return tube, a plasma collector, downstream tube leading from said process control to said plasma collector, a plasma supply container, and a plasma return tube leading from said plasma supply container to the patient for conveying uncontaminated plasma fluid to the patient.

2. The system of claim 1 wherein said separating structure comprises a centrifuge, and/or wherein preferably the collector is a dialyzer for removing contaminants from said plasma fluid and conveying purified plasma fluid to the patient.

3. The system of claim 1 including a first sterile connection device mounted at said upstream tube, a second sterile connection device mounted at said downstream tube, and a third sterile connection device mounted at said return tube, and/or wherein preferably said process control and said supply container comprise separate modules, which are selectively removable, and installable into said system by means of said sterile connection device.

4. The system of claim 1 including in combination therewith, a sterilizing assembly, said sterilizing assembly comprises a plurality of collection bags for containing contaminated fluid, such bag contents sterilized prior to dialyzing, a dialyzer, tubing connecting said collection container to with said dialyzer, a sterile connection device mounted at flow communication at said tubing for each of said collection container, a plurality of plasma supply containers communicating with said dialyzer for collecting sterilized plasma fluid from said dialyzer after contaminates have been removed from said plasma fluid, said supply containers being connected to said dialyzer by tubing, a sterile connection device for each of said supply containers mounted at said tubing, and one of said supply containers and one of said plasma collectors being said plasma collector and said supply container for said homodialysis system.

5. A plasma sterilizing assembly comprising a plurality of plasma collection containers for containing contaminated plasma, a dialyzer for removing contaminants from the plasma, inlet tubing connecting said collection containers with said dialyzer, a sterile connection device for each of said supply containers mounted at said supply tubing for selectively installing and removing each of said supply containers from said assembly.

6. A method of hemodialysis comprising mounting an upstream tube to a patient for the removal of whole blood containing red blood cells and white blood cells and plasma fluid from the patient, flowing the whole blood to a process control, separating the plasma fluid from the red blood cells by use of separating structure in the process control, flowing the plasma fluid to a plasma collector, returning the red blood cells and the white blood cells back to the patient, and supplying the patient with uncontaminated plasma from a supply container.

7. The method of claim 6 wherein the plasma fluid is separated from the cells by a centrifuge having a bowl which causes the cells to collect on the side of the centrifuge bowl while the plasma supply tubing for selectively installing and removing each of said supply containers from said assembly.

8. The method of claim 7 wherein viral elements are removed from the treated plasma to control viral proliferation.

9. The method of claim 8 wherein the method is used to reduce uncontrolled HIV spread within the patient to reduce HIV + becoming AIDS.

10. A hemodialysis system for removing substances from blood cells comprising a supply of blood cells containing substances, a removal station, said removal station having a spinning structure for receiving the blood cells and substances to separate the blood cells from the substances, and a collection station downstream from the removal station for collecting the substances.
